# FASCICULE DE BREVET EUROPEEN

(11) **EP 0 728 755 B1**
(45) Date de publication et mention de la délivrance du brevet: **11.04.2001**
(21) Numéro de dépôt: 96400359.4
(22) Date de dépôt: 22.02.1996
(51) Int. Cl.: C07D 333/24, A61K 31/38

(54) **Nouveaux composés du thiophène, leur procédé de préparation et les compositions pharmaceutiques les renfermant**
Thiophenverbindungen, Verfahren zu deren Herstellung und sie enthaltende pharmazeutische Zusammensetzungen
Thiophene compounds, process for their preparation and pharmaceutical compositions containing them

(30) Priorité: 23.02.1995 FR 9502061
(43) Date de publication de la demande: 28.08.1996
(73) Titulaire: ADIR ET COMPAGNIE, 92415 Courbevoie Cédex (FR)
(72) Inventeur: Wierzbicki, Michel, 78620 L'Etang la Ville (FR); Sauveur, Frédéric, 95100 Argenteuil (FR); Bonnet, Jacqueline, 75013 Paris (FR); Tordjman, Charles, 92100 Boulogne (FR)

(56) Documents cités:
- EP-A- 0 055 471
- EP-A- 0 429 370
- WO-A-91/19708
- FR-A- 2 588 871

## Description

La présente invention a pour objet les nouveaux composés du thiophène, leur procédé de préparation et les compositions pharmaceutiques les renfermant.

Elle concerne particulièrement les composés de formule I 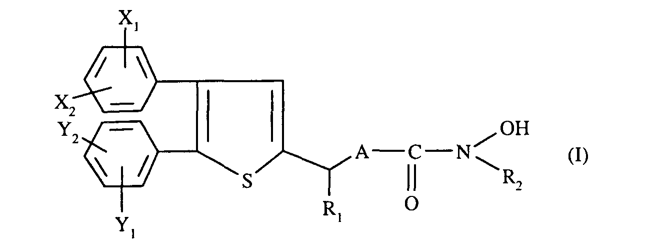 dans laquelle :
- X₁, X₂, Y₁ et Y₂, identiques ou différents, représentent chacun un atome d'hydrogène ou d'halogène, un radical alkyle ou alkoxy, ayant chacun de 1 à 5 atomes de carbone en chaîne droite ou ramifiée, ou un radical trifluorométhyle ;
- R₁ représente un atome d'hydrogène ou un radical alkyle ayant de 1 à 5 atomes de carbone en chaîne droite ou ramifiée ;
- A représente une chaîne hydrocarbonée droite de 1 à 5 atomes de carbone dont chaque atome de carbone est éventuellement mono-ou di- substitué par un radical alkyle ayant de 1 à 5 atomes de carbone, et
- R₂ représente un atome d'hydrogène, un radical alkyle ayant de 1 à 5 atomes de carbone en chaine droite ou ramifiée, un radical cyclohexyle ou un radical benzyle ; et leurs sels d'addition physiologiquement tolérables avec des bases appropriées.

L'état antérieur de la technique le plus proche de la présente invention est illustré notamment par la demande de brevet PCT 91/19708 qui concerne des composés de formule : 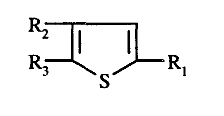 ayant une activité anti-inflammatoire,
laquelle référence ne décrit ni ne suggère les composés de formule I objet de la présente invention, lesquels composés présentent une activité pharmacologique et thérapeutique particulièrement intéressante dans le domaine de l'inflammation,
et par la demande de brevet FR 2 588 871 qui concerne des composés de formule : 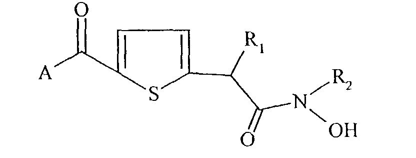 ayant des propriétés antiinflammatoire, antiasthmatique et immunomodulatrice.

La présente invention a également pour objet le procédé de préparation des composés de formule I caractérisé en ce que :
- l'on saponifie l'ester de formule II : 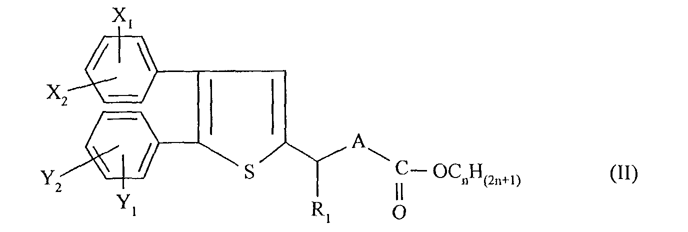 dans laquelle X₁, X₂, Y₁, Y₂, R₁ et A ont les significations précédemment définies,
   et n représente 1 ou 2,
- en acide de formule III : 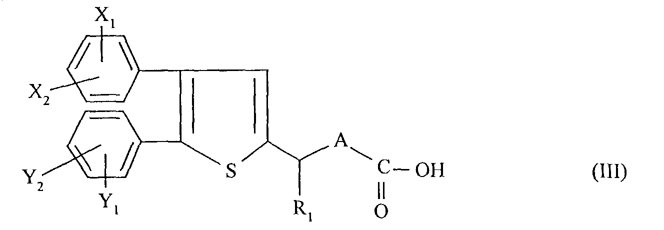 dans laquelle X₁, X₂, Y₁, Y₂, R₁ et A ont les significations précédemment définies,
- lequel acide est transformé en composé activé correspondant de formule IV : 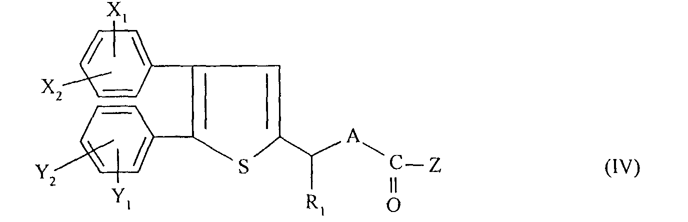 dans laquelle X₁, X₂, Y₁, Y₂, R₁ et A ont les significations précédemment définies, et
- Z représente un atome de chlore ou un radical imidazol-1-yle,
- sur lequel on fait réagir l'hydroxylamine O silylée de formule V : 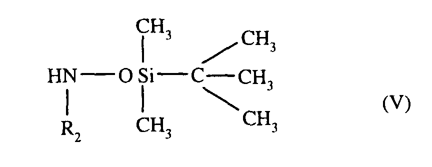 dans laquelle R₂ a la signification précédemment définie,
- pour obtenir le composé de formule VI : 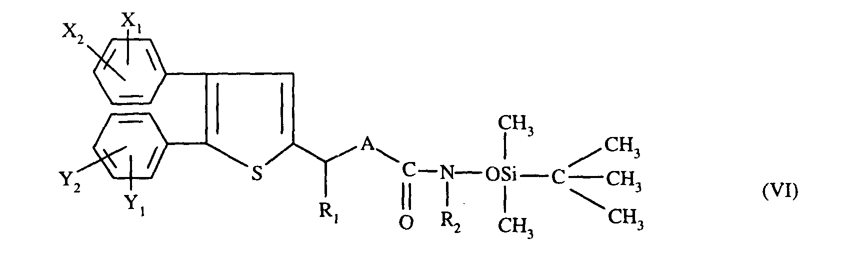 dans laquelle X₁, X₂, Y₁, Y₂, A, R₁ et R₂ ont les significations précédemment définies,
- que l'on hydrolyse pour obtenir le composé correspondant de formule I.

La saponification de l'ester de formule II est réalisée de façon adéquate au moyen de soude en milieu hydroalcoolique.

La transformation de l'acide de formule III en composé activé de formule IV s'effectue avantageusement soit en milieu chloroforme, au moyen de chlorure d'oxalyle pour obtenir les composés de formule IV dans laquelle Z représente un atome de chlore, soit en milieu dichlorométhane, au moyen de carbonyle diimidazole, pour obtenir les composés de formule IV dans laquelle Z représente un radical imidazol-1-yle.

La réaction des composés IV et V, pour conduire aux composés de formule VI est mise en oeuvre de façon particulièrement adéquate en opérant dans un solvant approprié tel que par exemple :
- l'acétonitrile, en présense de diméthylaminopyridine et de triéthylamine dans le cas où dans la formule du composé IV utilisé, Z représente un atome de chlore, et
- le dichlorométhane dans le cas où dans la formule du composé IV utilisé, Z représente un radical imidazol-1-yle.

L'hydrolyse du composé de formule VI, pour obtenir un composé de formule I, s'effectue avantageusement en opérant dans un mélange de dichlorométhane et d'acide trifluoroacétique.

L'ester de formule II, utilisé comme matière première, a été préparé selon le schéma opératoire suivant: 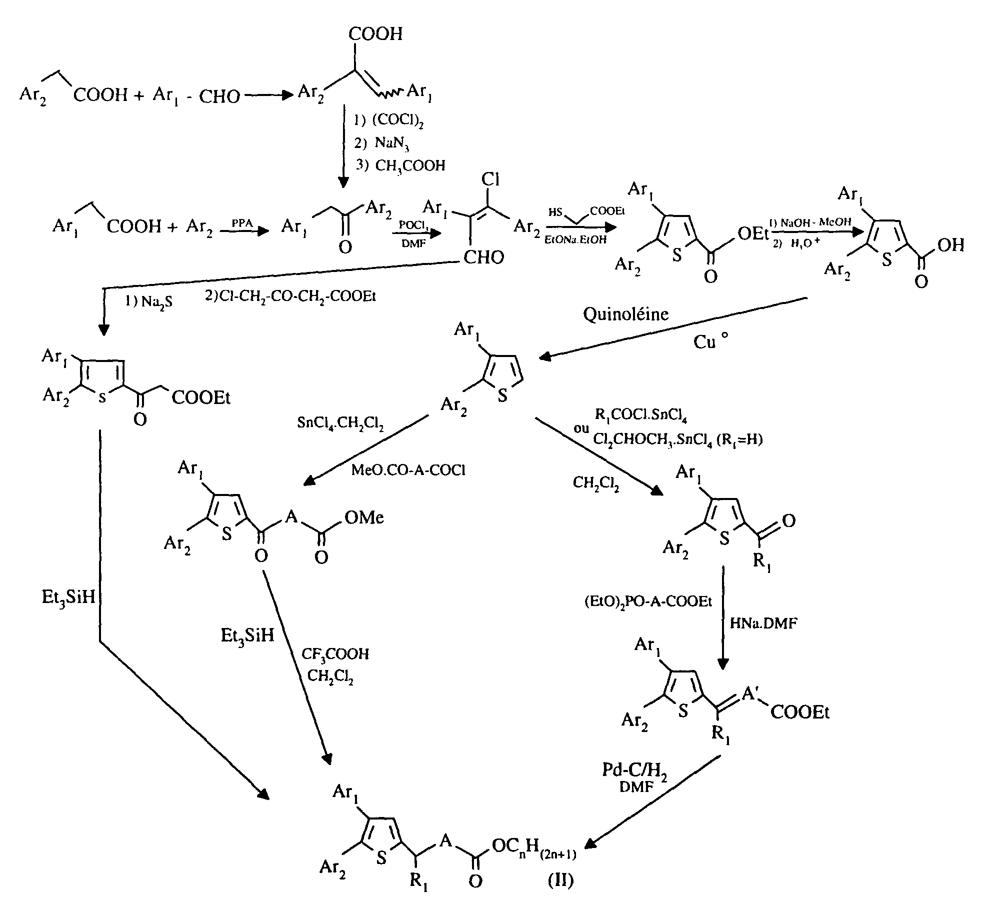 étant entendu que dans ce schéma :
Ar₁ représente : 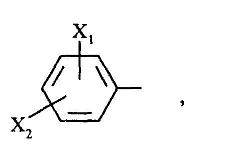
Ar₂ représente : 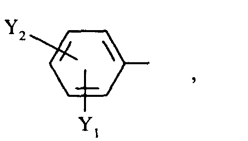
PPA = acide polyphosphorique,
DMF = diméthylformamide,
Et = -C₂H₅,
Me = -CH₃,
n = 1 ou 2,
R₁ et A ont les mêmes significations que dans la formule I, et
A' est tel que A = -HA'-

Les composés de formule I peuvent être transformés en sels d'addition physiologiquement tolérables, avec des bases appropriées. Préparés selon des méthodes classiques, comme décrit dans les exemples ci-après, ces sels font, à ce titre, partie de la présente invention.

Les composés de la présente invention possèdent des propriétés pharmacologiques et thérapeutiques intéressantes notamment dans le domaine de l'inflammation.

Au cours de l'inflammation, l'acide arachidonique, libéré des phospholipides de la membrane cellulaire par la phospholipase A2, est rapidement métabolisé par deux grandes voies enzymatiques : celle de la cyclooxygénase conduisant à la formation des prostaglandines et celle de la lipoxygénase conduisant à la formation des leucotriènes. Les prostaglandines -en particulier la PGE2- jouent un rôle important dans les phénomènes vasculaires liés à l'inflammation ainsi que dans la douleur inflammatoire tout en exerçant un effet protecteur sur la muqueuse gastrique. Deux types de cyclooxygénases ont été récemment mis en évidence : la cyclooxygénase constitutive de type 1 (COX1) se trouvant à l'état physiologique dans différents tissus dont l'estomac, et la cyclooxygénase de type 2 (COX2) induite lors des processus inflammatoires. Les leucotriènes -notamment le 5HETE et le leucotriène B4-puissants chémoattractants des neutrophiles, capables d'augmenter la perméabilité vasculaire et doués de propriétés immunologiques, contribuent à la destruction tissulaire progressive des maladies immunoinflammatoires telles que la polyarthrite rhumatoïde ; ils semblent par ailleurs impliqués dans les processus d'ulcération gastrique. L'inhibition de la cyclooxygénase par les anti-inflammatoires non stéroïdiens (AINS) est responsable de leur effet bénéfique sur les symptômes de la phase initiale de l'inflammation mais elle entraîne aussi les effets indésirables propres à cette classe de médicaments en particulier au niveau de la muqueuse gastrointestinale. Ces AINS restent sans effet sur la progression de la destruction tissulaire liée à la migration leucocytaire.

Les composés de la présente invention inhibent à la fois la voie enzymatique de la cyclooxygénase et celle de la lipoxygénase. De plus, ils exercent une inhibition préférentielle de la COX2 par rapport à la COX1. Ce profil particulier leur confère des effets anti-inflammatoires puissants s'exerçant en particulier sur les phénomènes de chronicité, et une tolérance gastrointestinale améliorée par rapport aux AINS classiques.

Les composés de la présente invention se révèlent ainsi de puissants agents anti-inflammatoires capables d'inhiber à la fois la phase vasculaire précoce et la phase chronique de destruction tissulaire liée à l'inflammation.

Leur effet thérapeutique s'exercera donc dans les inflammations rhumatologiques de nature aigüe ou chronique telles que par exemple la polyarthrite rhumatoïde et la spondylarthrite ankylosante, et dans les inflammations de type intestinal (maladie de Crohn) ou cutané (psoriasis) dans lesquelles se trouvent impliqués les leucotriènes.

La présente invention a également pour objet les compositions pharmaceutiques contenant comme principe actif un composé de formule I ou un de ses sels physiologiquement tolérable, mélangé ou associé à un excipient pharmaceutique approprié, comme par exemple le glucose, l'amidon, le talc, l'éthyl cellulose, le stéarate de magnésium, le beurre de cacao ou l'eau distillée.

Les compositions pharmaceutiques ainsi obtenues se présentent généralement sous forme dosée pouvant contenir de 10 à 300 mg de principe actif, et peuvent revêtir, par exemple, la forme de comprimés, dragées, gélules, suppositoires, solutions injectables ou buvables ou pommades, et être, selon les cas, administrées par voie orale, rectale, parentérale ou locale.

La posologie peut varier selon l'âge et le poids du patient, la voie d'administration, la nature de la maladie et les traitements associés.

A titre d'exemple, la posologie par voie orale peut s'échelonner de 10 à 300 mg de principe actif par jour.

Les exemples suivants illustrent la présente invention. Les points de fusion sont, sauf mention contraire, déterminés à la platine chauffante de Kofler.

### Exemple 1

### 3-[4,5-bis(4-méthoxyphényl)thién-2-yl] N-hydroxy N-méthyl propionamide.

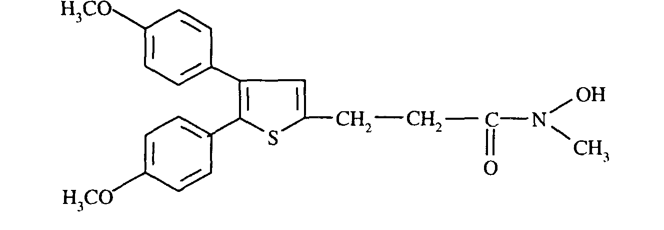

### A/ Préparation de l'acide 3-[4,5-bis(4-méthoxyphényl)thién-2-yl]propionique:

A 32,6 g (0,082 mole) de 3-[4,5-bis(4-méthoxyphényl)thién-2-yl]propionate d'éthyle et 94 ml d'éthanol, contenus dans un ballon tricol de 1 litre, on ajoute 94 ml de soude normale et porte l'ensemble à reflux pendant 1 heure. Après élimination de l'éthanol, la phase restante est reprise par 180 ml d'eau puis soumise à une extraction par 150 ml d'éther. La couche aqueuse est ensuite acidifiée avec 95 ml d'acide chlorhydrique normal puis soumise à une extraction avec 2 fois 300 ml d'éther.

Après séchage sur MgSO₄ et traitement au noir animal, puis filtration et concentration, on obtient 29 g de l'acide attendu sous forme d'un solide blanc fondant à 122 °C (Rendement : 96 %).

### B/ Préparation du composé titre.

Dans un ballon tricol de 1 litre muni d'une agitation mécanique, d'un thermomètre et d'une ampoule à brome, le tout placé dans un bain réfrigérant, on introduit 12 g (0,0746 mole) de N-méthyl O-[(Si-diméthyl-Si-tert-butyl)silyl], hydroxylamine, 240 ml d'acétonitrile, 0,9 g (0,0075 mole) de diméthylaminopyridine et 7,54 g (0,075 mole) de triéthylamine fraîchement rectifiée sur KOH. Le milieu réactionnel est amené à 0 °C dans un bain de saumure.

Pendant ce temps, on prépare le chlorure de 3-[4,5-bis(4-méthoxyphényl)thién-2-yl]propionyle, en faisant réagir 20,98 g de chlorure d'oxalyle sur 25 g (0,078 mole) de l'acide préparé au stade A en solution dans le chloroforme.

Après élimination de l'excès de chlorure d'oxalyle par distillation sous vide, le chlorure d'acide ainsi obtenu est mis en solution dans 50 ml de CH₃CN et la dite solution est ajoutée, en 20 minutes, dans le milieu réactionnel contenant la N-méthyl O-[(Si-diméthyl-Si-tert-butyl)silyl]hydroxylamine, tout en maintenant la température à 0 °C. Après quelques minutes d'agitation, la température est amenée à 20 °C puis le milieu est hydrolysé par 210 ml d'eau et dilué par 210 ml d'acétate d'éthyle. La phase organique est décantée et la phase aqueuse est extraite par deux fois 100 ml d'acétate d'éthyle.

Les phases organiques réunies sont lavées par 70 ml d'une solution saturée de NaCl, séchées sur MgSO₄, traitées au noir animal, filtrées et concentrées. L'huile brute obtenue (35,9 g, Rendement : 100 %) est utilisée sans purification.

Dans un ballon tricol de 1 litre muni d'une agitation magnétique, d'un thermomètre et d'une ampoule à brome, le tout placé dans un bain réfrigérant, on ajoute, en une heure, une solution de 35,9 g (0,0678 mole) du produit ci-dessus obtenu dans 280 ml de dichlorométhane, sur un mélange de 300 ml de dichlorométhane et 68,2 ml d'acide trifluoroacétique, tout en maintenant la température à 0 °C.

Puis la température est amenée à 20 °C et le milieu est hydrolysé par 300 ml d'eau. La phase organique décantée est lavée par 200 ml de bicarbonate à 10 % et 140 ml d'eau. Après séchage sur MgSO₄, traitement au noir animal, filtration et concentration, la phase obtenue est chromatographiée sur silice en éluant avec le mélange dichlorométhane/acétate d'éthyle (90/10). Les fractions utiles sont réunies, concentrées et le résidu obtenu cristallisé dans l'éther, essoré et séché donne 20,9 g du produit titre sous forme d'un solide cristallisé blanc fondant à 96 °C (Rendement : 78 %).

Le produit ainsi obtenu, traité par CaCl₂ et NaOH en milieu eau et méthanol donne le sel de calcium correspondant.

La même méthode, utilisant ZnCl₂ à la place de CaCl₂, a permis d'obtenir le sel de zinc correspondant.

### Exemples 2 à 29 :

En opérant comme décrit dans l'exemple 1, ont été préparés les composés objet des exemples regroupés dans le tableau suivant :

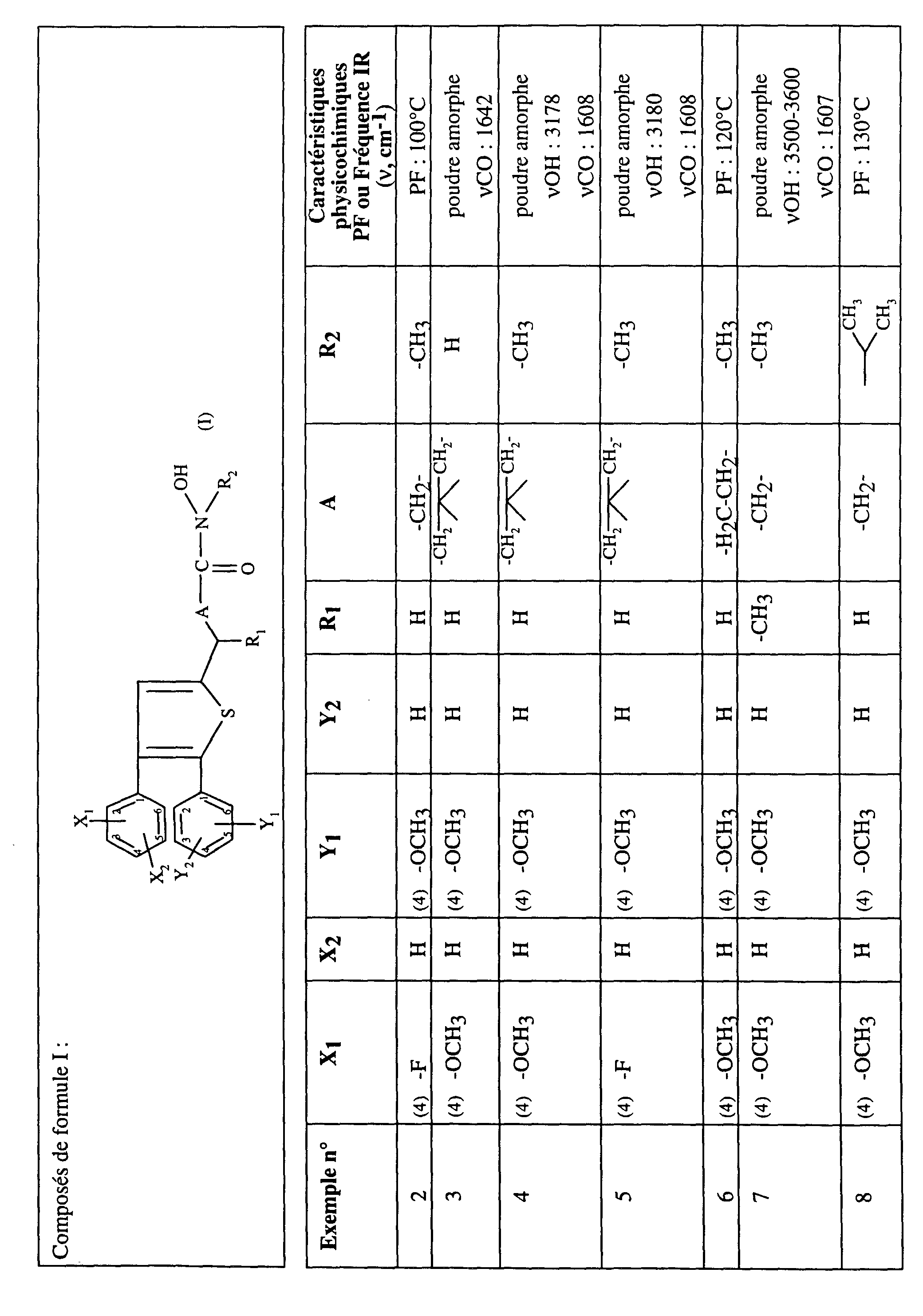

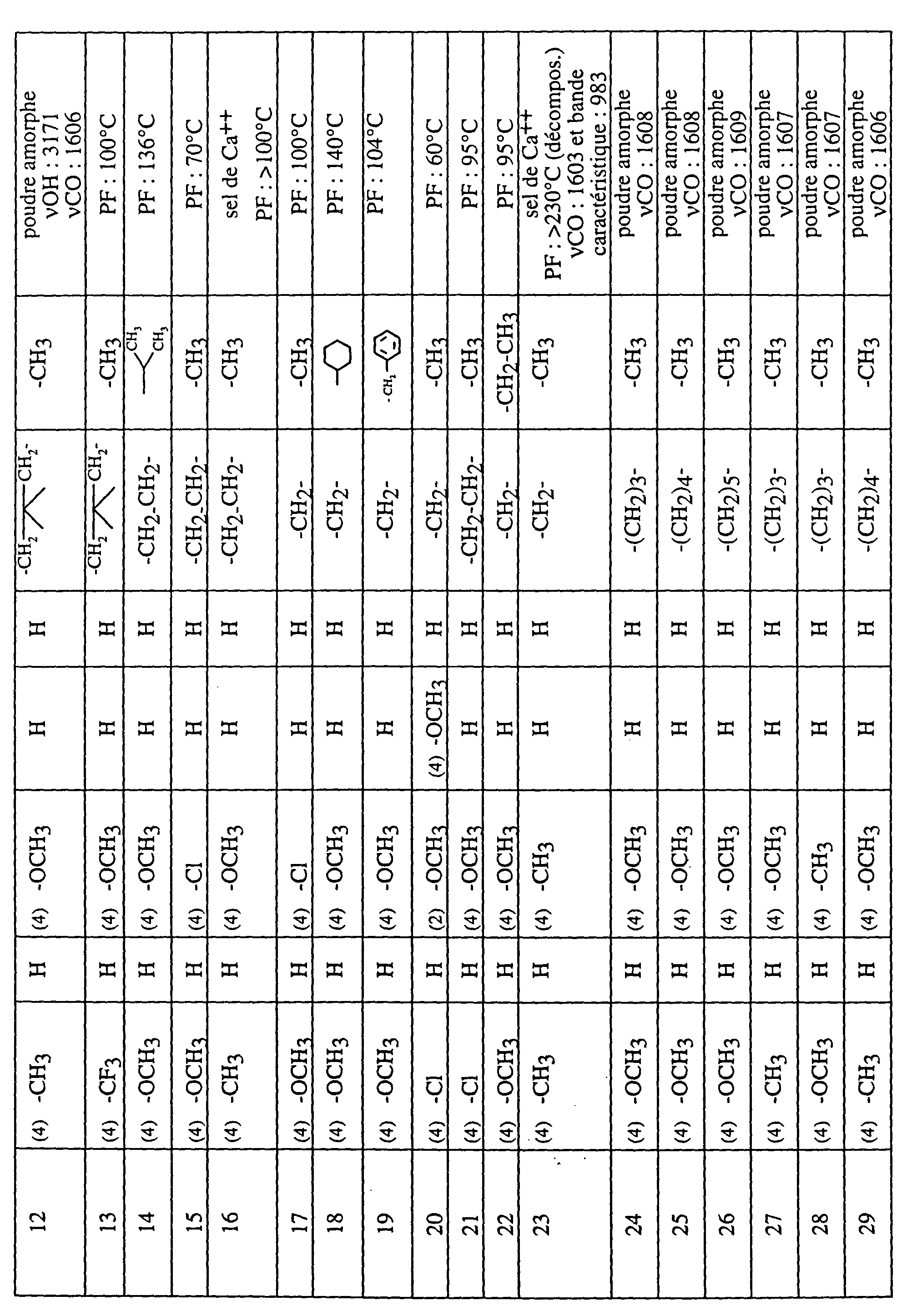

### Synthèse des matières premières de formule II :

Les esters de formule II utilisés comme matière première ont été synthétisés comme indiqué dans l'une ou l'autre des méthodes ci-après. Il est recommandé d'utiliser :
- la première méthode pour synthétiser les esters de formule II dans laquelle A représente - CH₂-, et
- la deuxième méthode dans les autres cas.

### Première méthode :

Elle a été utilisée pour préparer les esters II ayant servi de matières premières dans la synthèse des composés objet des exemples 1, 2, 7 à 11,17 à 20, 22 et 23.

Elle est exemplifiée, en détail, pour la préparation du 3-[4,5-bis-(4-méthoxyphényl)thién-2-yl]propionate d'éthyle, matière première utilisée pour la synthèse du composé objet de l'exemple 1.

### 1) Préparation du composé de formule :

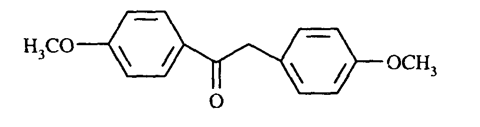

Dans un ballon tricol de 250 ml, muni d'une agitation mécanique et d'un thermomètre et plongé dans un bain d'huile, on verse 50 g d'acide polyphosphorique, 20 g (0,12 mole) d'acide 2-(4-méthoxyphényl)acétique et 15,2 g d'anisole. Le milieu réactionnel est porté à 120 °C sous agitation et maintenu à cette température pendant 45 minutes. Le milieu, qui devient rouge vif, est alors vivement versé sur un litre d'HCl 1N. L'ensemble est maintenu sous vive agitation durant 1 heure 30. Le précipité jaune formé est filtré, lavé abondamment à l'eau et soigneusement essoré. Le solide recueilli est dissous dans 600 ml de dichlorométhane. La phase organique est lavée par 200 ml d'eau, 100 ml d'une solution aqueuse de bicarbonate de sodium à 10 % et enfin 100 ml d'eau.

La phase organique, est séchée sur sulfate de magnésium, décolorée au noir animal, filtrée, concentrée et chromatographiée sur silice en éluant au CH₂Cl₂. Les fractions utiles sont réunies et concentrées. Le solide obtenu est repris à l'éther de pétrole, filtré, essoré et séché sous vide. On obtient 19,8 g du produit attendu sous forme d'un solide blanc, PF : 158 °C, Rendement : 64,5 %.

En opérant de la même façon, ont été préparés les composés ci-après :

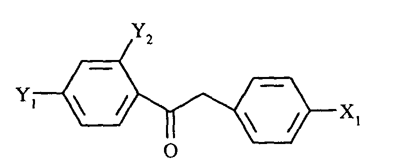

| **X**_{**1**} | **Y**_{**1**} | **Y**_{**2**} | **PF°C** |
|---|---|---|---|
| -F | -OCH₃ | H | 105 |
| -CH₃ | -OCH₃ | H | 92 |
| -CF₃ | -OCH₃ | H | 150 |
| -Cl | -OCH₃ | H | 135 |
| -Cl | -OCH₃ | -OCH₃ | 69 |

N.B. : Une variante de la méthode ci-dessus a été utilisée pour la préparation du composé de formule :

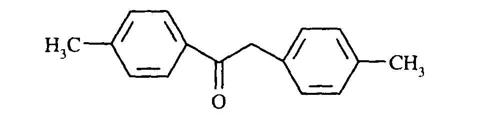

Dans un ballon tricol de 1 litre, on introduit 60 g d'acide paratolylacétique et 200 ml d'anhydride acétique. On ajoute à la suspension 48 g de paratolualdéhyde puis goutte à goutte 100 ml de triéthylamine. On porte à léger reflux pendant 12 heures puis on distille l'anhydride acétique. On reprend le mélange par 1 litre d'acétate d'éthyle, puis on ajoute lentement 1100 ml d'HCl (N). On décante, lave la phase organique par 100 ml d'HCl (N) puis 100 ml d'HCl (0,1 N). On concentre la phase organique en distillant 90 % de l'acétate d'éthyle. On filtre, lave le précipité avec un peu d'acétate d'éthyle glacé, puis à l'éther de pétrole. On obtient ainsi 45 g d'acide de formule :

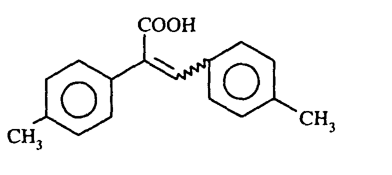

Cet acide est dissout dans 1 litre de chloroforme, on y additionne 68 g de chlorure d'oxalyle et chauffe à reflux jusqu'à cessation du dégagement gazeux. On distille ensuite le solvant et les substances volatiles. Le chlorure d'acide ainsi obtenu est dissout dans 200 ml d'acétone puis additionné lentement à un mélange de 37,4 g de bicarbonate de sodium et 12,7 g d'azidure de sodium dans 200 ml d'eau et 200 ml d'acétone. On laisse le milieu sous agitation à température ambiante pendant encore 1 heure puis distille l'acétone sous pression réduite. On extrait au toluène, en éliminant le résidu minéral formé, on lave à l'eau la phase organique, on la sèche au sulfate de magnésium puis on concentre à sec. On obtient ainsi 51 g d'une huile brune instable. On verse alors sur cette huile 230 ml d'acide acétique puis 125 ml d'eau, on porte au reflux pendant 1 heure puis refroidit et filtre le précipité formé qu'on lave à l'eau puis à l'éthanol froid. On obtient ainsi 35,6 g de composé de formule : 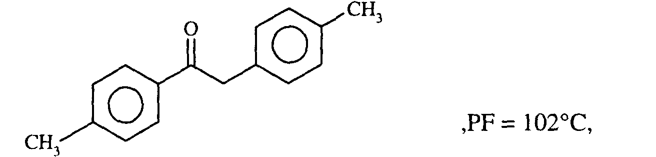 lequel servira pour préparer l'ester II, matière première pour la synthèse du composé objet de l'exemple 23.

De la même manière, a été préparé le composé de formule : 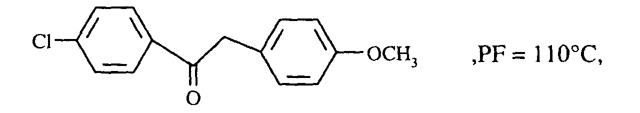 qui servira pour préparer l'ester II, matière première pour la synthèse des composés objet des exemples 15 et 17.

### 2) Préparation du composé de formule :

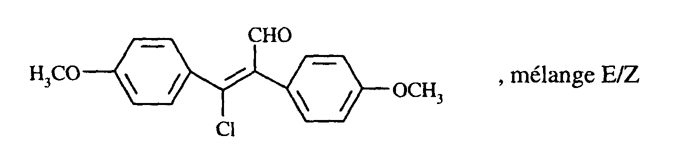

Dans un ballon tricol de 2 litres muni d'une agitation mécanique, d'un thermomètre et d'une ampoule à brome, placé dans un bain réfrigérant, on introduit 172 ml de diméthylformamide anhydre. Le milieu est amené à 0 °C grâce au bain de saumure, et on y ajoute 74,7 g (0,049 mole) de POCl₃ en trente minutes. Après agitation durant vingt minutes, 100 g (0,39 mole) de desoxyanisoïne en solution dans 700 ml de diméthylformamide anhydre sont additionnés sous agitation en 1 heure à une température entre 0 et 5 °C. Un précipité jaune apparaît rapidement. La température du milieu réactionnel est portée à 80 °C et maintenue ainsi pendant 6 heures. Le résidu est ensuite versé sur 750 ml d'une solution aqueuse d'acétate de sodium à 25 %. Le précipité formé est filtré, lavé à l'eau, et malaxé dans 1 litre d'éthanol bouillant. Après refroidissement de la suspension, filtration, essorage et séchage du produit, on obtient 104 g du produit attendu sous forme d'un solide blanc, fondant à 158 °C (Rendement : 88 %).

En opérant de la même façon, ont été préparés les composés ci-après, sous forme de mélange E/Z :

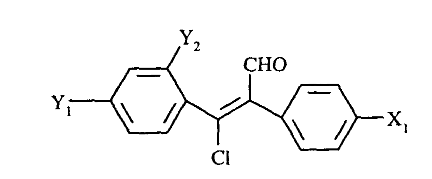

| **X**_{**1**} | **Y**_{**1**} | **Y**_{**2**} | **PF°C** |
|---|---|---|---|
| -F | -OCH₃ | H | 128 |
| -CH₃ | -OCH₃ | H | 126 |
| -CF₃ | -OCH₃ | H | 125 |
| -Cl | -OCH₃ | H | 126 |
| -Cl | -OCH₃ | -OCH₃ | 128 |
| -OCH₃ | -Cl | H | 113 |
| -CH₃ | -CH₃ | H | 145 |

### 3) Préparation du composé de formule :

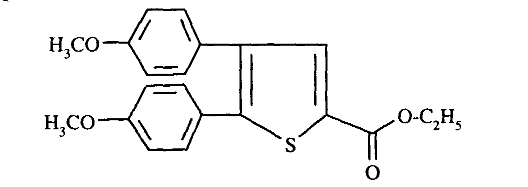

Dans un ballon tricol de 2 litres muni d'une agitation mécanique, d'un thermomètre, d'une ampoule de coulée et plongé dans un bain d'huile, on verse 450 ml d'éthanol anhydre, dans lesquels on ajoute peu à peu 6,7 g (0,29 mole) de sodium. Le milieu est alors porté à reflux jusqu'à dissolution complète. Après refroidissement, 28 g (0,231 mole) de 2-mercapto acétate d'éthyle en solution dans 100 ml d'éthanol anhydre sont rapidement ajoutés. La solution incolore obtenue est refroidie à 0 °C sur saumure. Le dérivé chloro formylé préparé au paragraphe 2) ci-dessus est alors ajouté par fraction en 30 minutes. Le milieu est agité pendant 4 heures, puis amené à température ambiante. Un fin précipité apparaît. Après distillation de l'éthanol, le résidu est repris par 650 ml d'éther anhydre et glacé 48 heures. Le chlorure de sodium est filtré et la solution éthérée est concentrée. Le résidu reprécipité dans 100 ml d'isopropanol donne 72 g du produit attendu sous forme d'un solide jaune fondant à 75 °C (Rendement : 84 %).

En opérant de la même façon, ont été préparés les composés ci-après :

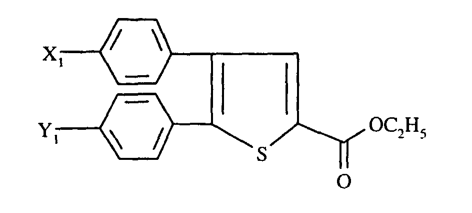

| **X**_{**1**} | **Y**_{**1**} | **PF°C** |
|---|---|---|
| -F | -OCH₃ | 120 |
| -CH₃ | -OCH₃ | 115 |
| -CF₃ | -OCH₃ | 88 |
| -Cl | -OCH₃ | 95 |
| -OCH₃ | -Cl | 90 |
| -CH₃ | -CH₃ | 76 |

### 4) Préparation du composé de formule

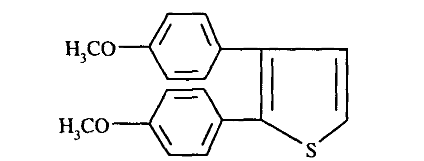

Dans un ballon monocol de 1 litre muni d'un réfrigérant et d'une agitation mécanique, 71,7 g (0,194 mole) de l'ester précédemment préparé sont mis en suspension dans 197 ml d'éthanol et 197 ml de soude 1N. Le milieu est porté au reflux et agité pendant 1 heure. Le solide passe rapidement en solution. L'éthanol est distillé sous pression réduite. Le résidu est repris par 200 ml d'eau. La phase aqueuse lavée par 3 fois 100 ml d'éther est acidifiée par 50 ml d'HCl 4N. Le précipité jaune obtenu est filtré, lavé à l'eau, puis quelques ml d'éthanol glacé, quelques ml d'éther et enfin à l'éther de pétrole. Après séchage à l'air, on obtient 51 g d'un solide blanc, qui est l'acide [4,5-bis(4-méthoxyphényl)thién-2-yl]carboxylique, PF : 215 °C (Rendement : 77 %).

30 g de ce solide sont mis en suspension dans 180 ml de quinoléine additionnée de 1 g de cuivre en poudre. Le milieu est chauffé sur bain d'huile jusqu'à 180 °C et maintenu à cette température jusqu'à fin du dégagement gazeux, soit 45 minutes. Après refroidissement, le résidu est versé sur un mélange de 520 g d'eau et 325 ml d'HCl concentré. La phase aqueuse est extraite par 3 fois 650 ml d'éther. La phase éthérée est lavée par 200 ml d'eau, 200 ml de bicarbonate de sodium aqueux à 10 % puis 200 ml d'eau.

Après traitement à MgSO₄ et au noir animal, la phase organique est concentrée et le résidu cristallisé dans 100 ml d'éther de pétrole est filtré, essoré et séché à l'air. On obtient 24,7 g du produit attendu, sous forme d'un solide blanc, fondant à 113-114 °C (Rendement : 95 %).

En opérant de la même façon, ont été préparés les composés ci-après :

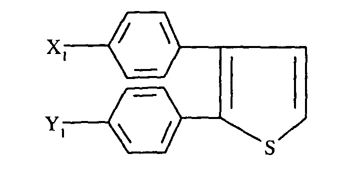

| **X**_{**1**} | **Y**_{**1**} | **PF°C** |
|---|---|---|
| -F | -OCH₃ | 80 |
| -CH₃ | -OCH₃ | 60 |
| -CF₃ | -OCH₃ | 88 |
| -Cl | -OCH₃ | 96 |
| -OCH₃ | -Cl | 110 |
| -CH₃ | -CH₃ | 85 |

à partir, respectivement, des acides intermédiaires suivants :

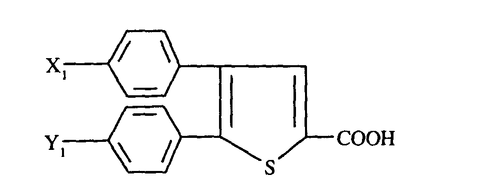

| **X**_{**1**} | **Y**_{**1**} | **PF°C** |
|---|---|---|
| -F | -OCH₃ | 185 |
| -CH₃ | -OCH₃ | 234 |
| -CF₃ | -OCH₃ | 206 |
| -Cl | -OCH₃ | 212 |
| -OCH₃ | -Cl | 218 |
| -CH₃ | -CH₃ | 264 |

### 5) Préparation du composé de formule :

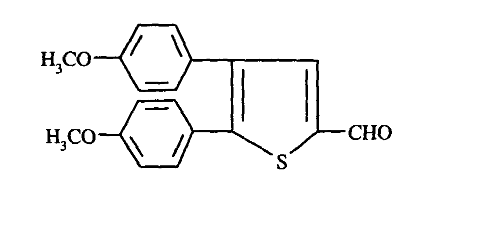

Dans un ballon tricol de 2 litres muni d'une agitation mécanique, d'un thermomètre, d'une ampoule à brome et placé sous courant d'azote, on charge 29,6 g (0,0998 mole) du 2,3-bis(4-méthoxyphényl)thiophéne [préparé comme au paragraphe 4)] en solution dans 450 ml de dichlorométhane anhydre. Le milieu est amené à - 50 °C et 13,3 g (0,115 mole) de dichlorométhyl éther sont ajoutés en une fois et 35,3 g (0,135 mole) de SnCl₄ sont alors additionnés en 20 minutes à - 50°C. (Une coloration brune apparait). La température est ramenée à 20°C et le milieu est hydrolysé sur un mélange eau-glace (150 g) -HCl concentré (25 ml). La phase organique est décantée et la phase aqueuse extraite par 2 fois 50 ml de dichlorométhane. Les phases organiques réunies sont lavées par 60 ml d'HCl 1N, 60 ml d'eau, 60 ml de bicarbonate de sodium et enfin 60 ml d'eau, puis traitées par MgSO₄ et noir animal avant d'être concentrées. Le résidu est cristallisé dans 80 ml d'éther ; le précipité est filtré, essoré et séché à l'air. On obtient ainsi 28,5 g du produit attendu, sous forme d'un solide blanc, fondant à 122°C (Rendement : 88 %).

En opérant de la même façon ont été préparés les composés ci-après :

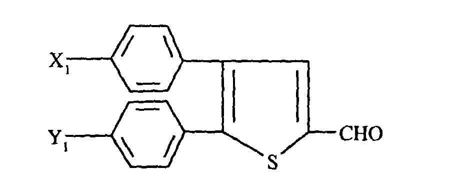

| **X**_{**1**} | **Y**_{**1**} | **PF°C** |
|---|---|---|
| -F | -OCH₃ | amorphe |
| -CH₃ | -OCH₃ | 112 |
| -CF₃ | -OCH₃ | 130 |
| -Cl | -OCH₃ | 128 |
| -OCH₃ | -Cl | 105 |
| -CH₃ | -CH₃ | 85 |

### 6) Préparation du composé de formule :

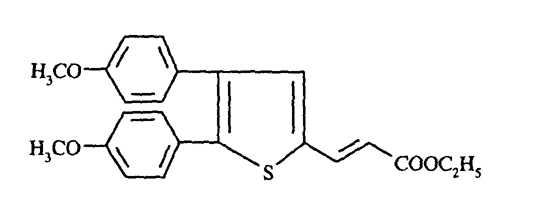

Dans un ballon tricol de 1 litre, muni d'une agitation mécanique, d'un thermomètre et d'une ampoule à brome, on met en suspension 3,85 g (0,0963 mole) d'hydrure de sodium dans 63 ml de diméthylformamide anhydre, puis on ajoute 21,6 g (0,0963 mole) de triéthylphosphonoacétate en solution dans 182 ml de diméthylformamide anhydre en 40 minutes. La réaction est exothermique. Après 30 minutes d'agitation, la température est amenée à 20 °C et 28,5 g (0,0878 mole) d'aldéhyde en solution dans 103 ml de diméthylformamide anhydre sont ajoutés en 20 minutes. Le milieu est maintenu sous agitation pendant 10 heures (solution orangée).
Le diméthylformamide est distillé sous pression réduite. Le résidu est repris dans 120 ml d'eau, puis extrait par 3 fois 300 ml d'éther. La phase organique est séchée sur MgSO4, traitée au noir animal, filtrée et concentrée. On obtient 33,6 g du produit attendu, sous forme d'un solide blanc fondant à 88-90 °C (Rendement : 97 %).

En opérant de la même façon, ont été préparés les composés ci-après :

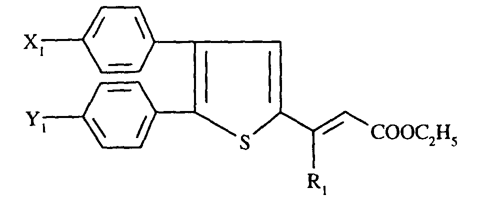

| **R**_{**1**} | **X**_{**1**} | **Y**_{**1**} | **PF °C** |
|---|---|---|---|
| H | -F | -OCH₃ | 118 |
| H | -CH₃ | -OCH₃ | 87 |
| H | -CF₃ | -OCH₃ | 123 |
| H | -Cl | -OCH₃ | amorphe |
| (*) CH₃ | -OCH₃ | -OCH₃ | amorphe |
| H | -OCH₃ | -Cl | huile brute utilisée sans purification |
| H | -CH₃ | -CH₃ | huile brute utilisée sans purification |

| | | | |
|---|---|---|---|
| (*) NB : Ce composé a été préparé à partir du 2,3-bis-(4-méthoxyphényl)thiophène substitué en 5 par le radical -COCH₃, lui-même préparé selon la méthode décrite au paragraphe 5) ci-dessus. | | | |

### Préparation des composés de formule :

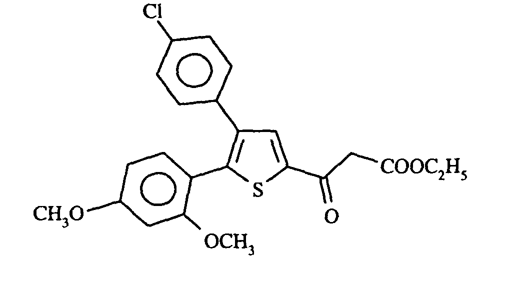

Dans un tricol de 500 ml, on introduit 11,9 g de sulfure de sodium monohydrate et 50 ml de diméthylformamide. On ajoute 5 gouttes de triéthylamine, puis 15,2 g de 2-(4-chlorophényl) 2-(α-chloro 2,4-diméthoxybenzylidène) acétaldéhyle en solution dans 100 ml de diméthylformamide. On laisse agiter pendant 1 heure à température ambiante. On refroidit alors à -10°C. On additionne 7,4 g de 4-chloroacétylacétate d'éthyle en solution dans 13 ml de diméthylformamide. On agite à -10°C pendant 1 heure puis à température ambiante pendant 24 heures. On verse l'ensemble sur 1 litre de solution saturée de NaCl. On filtre le solide gommeux formé. On reprend au dichlorométhane et chromatographie sur silice en éluant au dichlorométhane. On obtient ainsi 8,5 g d'un produit pur à ≈ 95 %.

### 7) Préparation du 3-[4,5-bis-(4-méthoxyphényl)thién-2-yl]propionate d'éthyle :

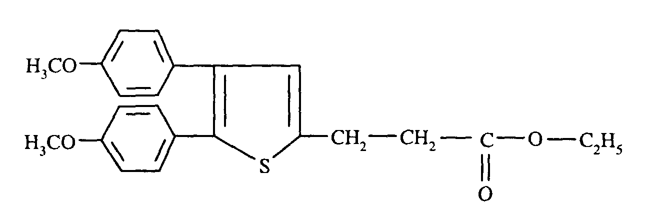

(matière première utilisée dans l'exemple 1) :
33,6 g (0,085 mole) du composé éthylénique préparé au paragraphe 6) sont mis en solution dans 900 ml de diméthylformamide anhydre et chargés dans un hydrogénateur basse pression. On y ajoute 9 g de charbon palladié à 10 % de Pd. Après plusieurs purges, l'hydrogène est introduit sous 5,5.10⁵ Pa et le milieu réactionnel est agité vivement. Lorsque l'hydrogène n'est plus absorbé, la réaction est continuée pendant 15 minutes à 50 °C. Après refroidissement, la solution est filtrée, le diméthylformamide est éliminé sous vide et le résidu mis en solution dans 20 ml d'un mélange éther/éther de pétrole (50/50). Après filtration sur papier, les solvants sont distillés. On obtient 32,6 g du produit attendu sous forme d'une huile jaune pâle. (Rendement: 96,7 %).

En opérant de la même façon, ont été préparés les composés ci-après :

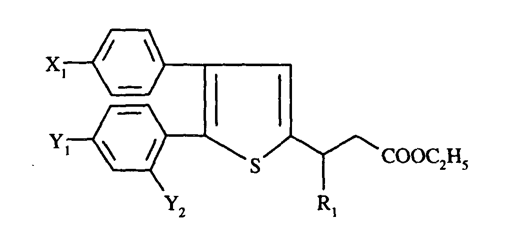

| **R**_{**1**} | **X**_{**1**} | **Y**_{**1**} | **Y**_{**2**} | **I.R. νCO (cm**^{**-1)**} |
|---|---|---|---|---|
| H | -F | -OCH₃ | H | 1733 |
| H | -CH₃ | -OCH₃ | H | 1734 |
| H | -CF₃ | -OCH₃ | H | 1735 |
| H | -Cl | -OCH₃ | H | 1734 |
| CH₃ | -OCH₃ | -OCH₃ | H | 1733 |
| H | -OCH₃ | -Cl | H | 1733 |
| H | -CH₃ | -CH₃ | H | 1735 |
| H | -Cl | -OCH₃ | -OCH₃ | 1734 |

### Seconde méthode

Elle a été utilisée pour préparer les esters II utilisés comme matières premières dans la synthèse des composés objet des exemples 3 à 6, 12 à 16 et 21.

A titre d'exemple, elle est décrite ci-après en détail pour la préparation du 5-[4,5-bis(4-méthoxyphényl)thién-2-yl]3,3-diméthyl pentanoate de méthyle, matière première utilisée pour la synthèse du composé objet de l'exemple 3.

### 1) Préparation du composé de formule :

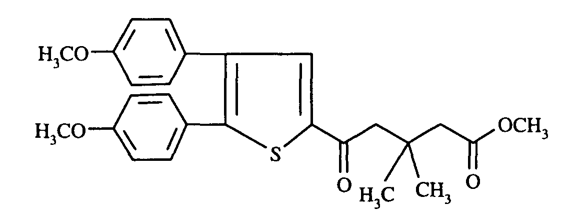

Dans un ballon tricol de 250 ml muni d'une agitation magnétique, d'un thermomètre, d'une ampoule à brome et placé dans un bain réfrigérant, on introduit 3 g (0,0101 mole) de 2,3-bis(4-méthoxyphényl)thiophène [préparé comme décrit dans la première méthode paragraphe 4)], en solution dans 32 ml de dichlorométhane anhydre. Le milieu est refroidi à 0 °C sur saumure, puis on y ajoute le chlorure de 3,3-diméthyl glutarate de méthyle préparé à partir de 1,75 g (0,01 mole) de 3,3-diméthyl glutarate de méthyle et de 2 ml de chlorure de thionyle. On y ajoute alors, en 10 minutes, 39 g (0,015 mole) de SnCl₄ (une coloration rouge apparaît). Après 30 minutes sous agitation, le résidu est hydrolysé sur un mélange eau-glace (20 g) et HCl concentré (2 ml). La phase aqueuse est extraite par 3 fois 30 ml de dichlorométhane. La phase organique est lavée par 20 ml d'HCl, 1N, 20 ml de bicarbonate de sodium aqueux à 10 %, et enfin 20 ml d'eau. Après traitement par MgSO₄ et noir animal, filtration et concentration, on obtient 4,7 g du produit attendu sous forme d'une huile incolore (Rendement : 100 %).

En opérant de la même façon, ont été préparés les composés suivants :

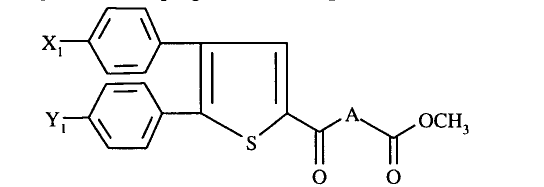

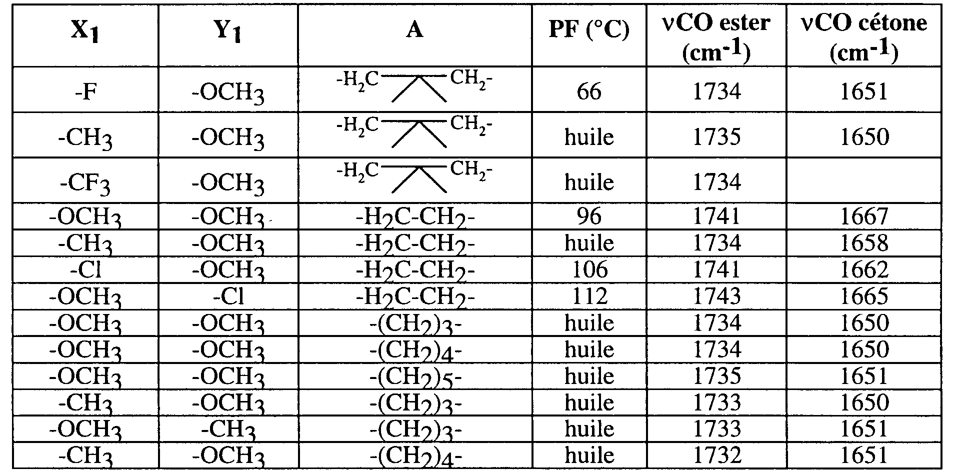

### 2) Préparation du 5-[4,5-bis(4-méthoxyphényl)thién-2-yl]3,3-diméthyl pentanoate de méthyle :

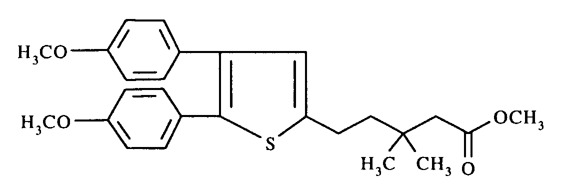

(utilisé comme matière première pour la synthèse des composés objet des exemples 3 et 4). Dans un ballon tricol de 100 ml équipé d'une agitation magnétique, d'un réfrigérant et d'un thermomètre, on ajoute 5,94 g (0,0131 mole) du composé préparé comme décrit ci-dessus au paragraphe 1) et 5,98 g (0,052 mole) d'acide trifluoroacétique à 10 ml de dichlorométhane anhydre. 3,04 g (0,0262 mole) de triéthylsilane sont ensuite additionnés en 10 minutes. Le milieu est porté au reflux pendant 8 heures et de nouveau 3,04 g de triéthylsilane sont ajoutés. Après 10 heures de reflux supplémentaire, le résidu est concentré, repris par 20 ml de dichlorométhane, lavé par 20 ml d'une solution aqueuse saturée de bicarbonate de sodium, puis 20 ml d'eau.

Après traitement par MgSO4 et le noir animal, filtration et chromatographie sur colonne de silice en éluant au dichlorométhane, la concentration des fractions utiles laisse 17 g du produit attendu sous forme d'une huile incoloree (Rendement : 67 %).

En opérant de la même façon, ont été préparés les composés suivants :

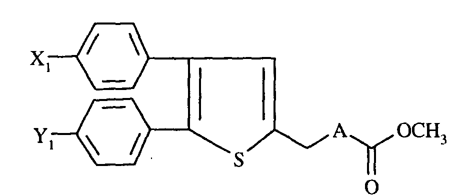

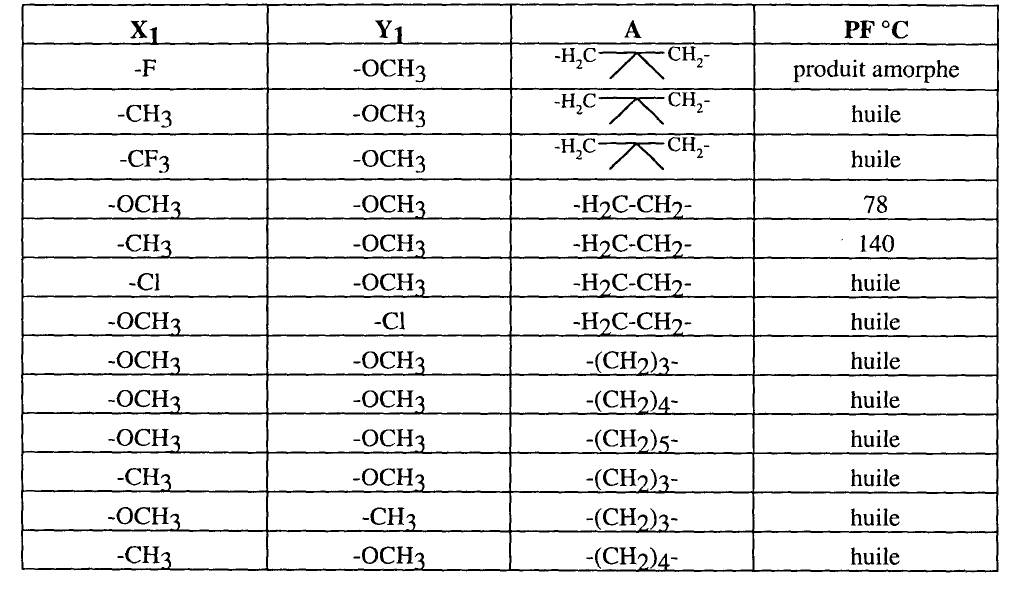

### Exemple 30

### Etude pharmacologique

1) L'activité des composés de l'invention sur la biosynthèse des médiateurs de la voie de la cyclooxygénase d'une part et de celle de la lipoxygénase d'autre part a été mise en évidence in vitro sur polymorphonucléaires de rat stimulés par l'ionophore A23187, selon le protocole décrit par C. Tordjman and coll. ("High-performance liquid chromatographic quantitation of cyclooxygenase and lipoxygenase metabolites of arachidonic acid from rat polymorphonuclear leukocytes" Journal of Chromatography, 532, 1990, 135-143).
Les résultats obtenus sont rassemblés dans le tableau ci-après :

| **COMPOSES** | **IC50 (µm)** | |
|---|---|---|
| | Voie de la cyclooxygénase (PGE2) | voie de la lipoxygénase (LTB4) |
| Exemple 1 | 0,05 | 0,03 |
| Exemple 2 | 0,38 | 0,08 |
| Exemple 3 | 2,50 | 1,00 |
| Exemple 4 | 0,75 | 0,38 |
| Exemple 5 | 2,50 | 0,25 |
| Exemple 6 | 0,38 | 0,05 |
| Exemple 16 | 0,05 | 0,05 |
| Exemple 22 | 0,08 | 0,05 |

2) L'activité in vivo des composés de l'invention a été étudié sur un test de douleur inflammatoire aigüe chez la souris - test de Siegmund ("Screening of analgesics, including aspirin-type compounds, based upon the antagonism of chemically induced "writhing" in mice" J. Pharm. Exp. Ther. 119, 1957, 184). Par exemple, après administration orale des composés, une inhibition de 50 % des réactions de l'animal a été provoquée par une dose de 1 mg/kg pour le composé de l'exemple 1, de 2,5 mg/kg pour le composé de l'exemple 6 et de 5 mg/kg pour le composé de l'exemple 2.
3) L'activité sur l'inflammation chronique a été évaluée sur un modèle autoimmun de polyarthrite à l'adjuvant de Freund chez le rat selon le protocole décrit par J. Bonnet et coll. ("Bone morphometric changes in adjuvant-induced polyarthritic osteopenia in rats : evidence for an early bone formation defect" J. Bone Miner. Res. 8, 1993, 659-668). A titre d'exemple, le composé de l'exemple 1, à la dose orale de 5 mg/kg/j, inhibe de 30 % la diffusion de l'arthrite au 14ème jour de la pathologie.
4) Conclusion : Les résultats ci-dessus montrent que les composés testés sont de puissants agents antiinflammatoires capables d'inhiber à la fois la phase vasculaire précoce et la phase chronique de destruction tissulaire liée à l'inflammation. Ils pourront donc de ce fait être utilisés dans le traitement des inflammations rhumatologiques de nature aigüe ou chronique (polyarthrite rhumatoïde, spondylarthrite ankylosante), et des inflammations de type intestinal (maladie de Crohn) ou cutané (psoriasis) dans lesquelles se trouvent impliqués les leucotriènes.

## Revendications

1. Les composés du thiophène de formule I 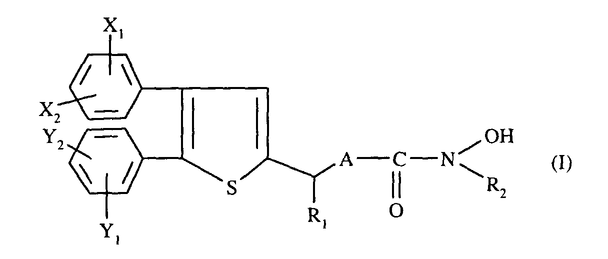 dans laquelle :
- X₁, X₂, Y₁ et Y₂, identiques ou différents, représentent chacun un atome d'hydrogène ou d'halogène, un radical alkyle ou alkoxy, ayant chacun de 1 à 5 atomes de carbone en chaîne droite ou ramifiée, ou un radical trifluorométhyle ;
- R₁ représente un atome d'hydrogène ou un radical alkyle ayant de 1 à 5 atomes de carbone en chaîne droite ou ramifiée ;
- A représente une chaîne hydrocarbonée droite de 1 à 5 atomes de carbone dont chaque atome de carbone est éventuellement mono-ou di- substitué par un radical alkyle ayant de 1 à 5 atomes de carbone, et
- R₂ représente un atome d'hydrogène, un radical alkyle ayant de 1 à 5 atomes de carbone en chaine droite ou ramifiée, un radical cyclohexyle ou un radical benzyle ;
et leurs sels d'addition physiologiquement tolérables avec des bases appropriées.

2. Un composé de la revendication 1 qui est le 3-[4,5-bis-(4-méthoxyphényl)thién-2-yl] N-hydroxy N-méthyl propionamide et son sel de calcium.

3. Un composé de la revendicaion 1 qui est le 3-[4-(4-fluorophényl)-5-(4-méthoxyphényl)thién-2-yl] N-hydroxy N-méthylpropionamide.

4. Un composé de la revendication 1 qui est le 4-[4,5-bis-(4-méthoxyphényl)thién-2-yl] N-hydroxy N-méthylbutanamide.

5. Un composé de la revendication 1 qui est le 3-[4-(4-méthylphényl)-5-(4-méthoxyphényl)thién-2-yl] N-hydroxy N-méthylpropionamide et son sel de calcium.

6. Un composé de la revendication 1 qui est le 3-[4,5-bis-(4-méthoxyphényl)thién-2-yl] N-éthyl N-hydroxypropionamide.

7. Le procédé de préparation des composés de la revendication 1 caractérisé en ce que :
- l'on saponifie l'ester de formule II : 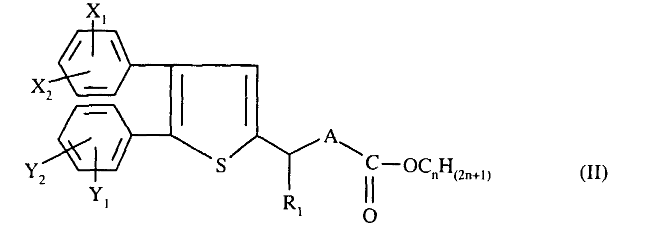 dans laquelle X₁, X₂, Y₁, Y₂, R₁ et A ont les significations définies dans la revendication 1, et n représente 1 ou 2
- en acide de formule III : 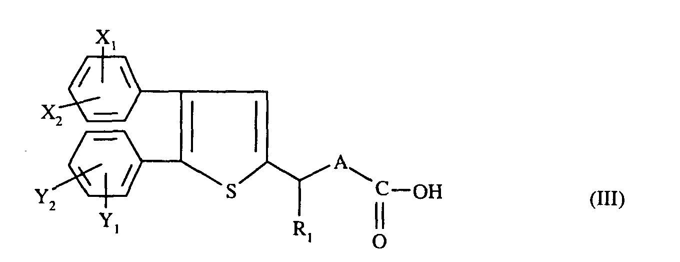 dans laquelle X₁, X₂, Y₁, Y₂, R₁ et A ont les significations précédemment définies,
- lequel acide est transformé en composé activé correspondant de formule IV : 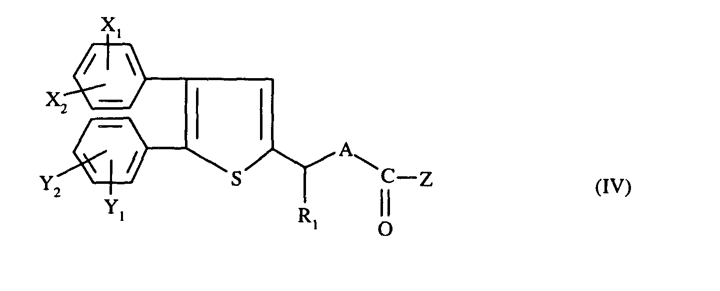 dans laquelle :
- X₁, X₂, Y₁, Y₂, R₁ et A ont les significations précédemment définies, et
- Z représente un atome de chlore ou un radical imidazol-1-yle,
- sur lequel on fait réagir l'hydroxylamine O silylée de formule V : 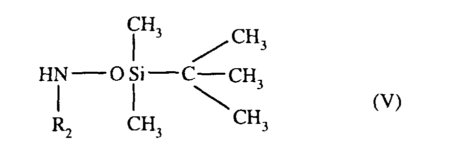 dans laquelle R₂ a la signification définie dans la revendication 1,
- pour obtenir le composé de formule VI : 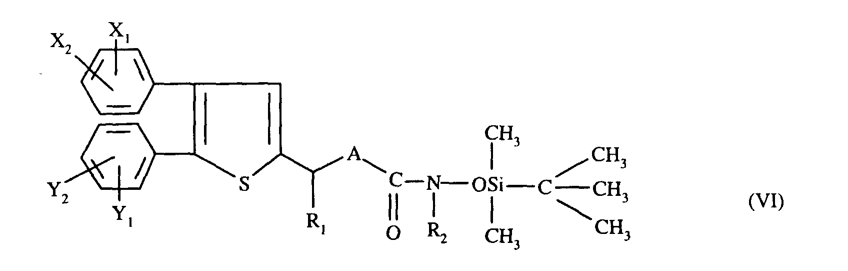 dans laquelle X₁, X₂, Y₁, Y₂, A, R₁ et R₂ ont les significations précédemment définies,
- que l'on hydrolyse pour obtenir le composé correspondant de formule I,
- et si on le désire, on transforme les composés ainsi obtenus en leurs sels d'addition avec des bases pharmaceutiquement acceptables.

8. A titre de médicament, un composé selon l'une quelconque des revendications 1 à 6.

9. Les compositions pharmaceutiques, utilisables dans le traitement des inflammations rhumatologiques de nature aigüe ou chronique et des inflammations de type intestinal ou cutané dans lesquelles se trouvent impliqués les leucotriènes, contenant comme principe actif au moins un des composés selon l'une quelconque des revendications 1 à 6, seul ou en combinaison avec un ou plusieurs excipients pharmaceutiques appropriés.

## Patentansprüche

1. Thiophenverbindungen der Formel I 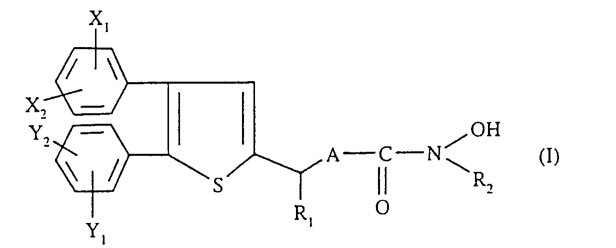 in der:
- X₁, X₂, Y₁ und Y₂, die gleichartig oder verschieden sind, jeweils ein Wasserstoffatom oder ein Halogenatom, eine Alkyl- oder Alkoxygruppe mit jeweils 1 bis 5 Kohlenstoffatomen in gerader oder verzweigter Kette, oder eine Trifluormethylgruppe;
- R₁ ein Wasserstoffatom oder eine Alkylgruppe mit 1 bis 5 Kohlenstoffatomen in gerader oder verzweigter Kette;
- A eine geradkettige Kohlenwasserstoffkette mit 1 bis 5 Kohlenstoffatomen, bei der jedes Kohlenstoffatom gegebenenfalls durch eine Alkylgruppe mit 1 bis 5 Kohlenstoffatomen mono- oder disubstituiert ist, und
- R₂ ein Wasserstoffatom, eine Alkylgruppe mit 1 bis 5 Kohlenstoffatomen in gerader oder verzweigter Kette, eine Cyclohexylgruppe oder eine Benzylgruppe bedeuten;
und deren physiologisch verträgliche Additionssalze mit geeigneten Basen.

2. Verbindung nach Anspruch 1, nämlich 3-[4,5-Bis-(4-methoxyphenyl)-thien-2-yl]-N-hydroxy-N-methyl-propionamid und dessen Calciumsalz.

3. Verbindung nach Anspruch 1, nämlich 3-[4-(4-Fluorphenyl)-5-(4-methoxyphenyl)-thien-2-yl]-N-hydroxy-N-methylpropionamid.

4. Verbindung nach Anspruch 1, nämlich 4-[4,5-Bis-(4-methoxyphenyl)-thien-2-yl]-N-hydroxy-N-methylbutanamid.

5. Verbindung nach Anspruch 1, nämlich 3-[4-(4-Methylphenyl)-5-(4-methoxyphenyl)-thien-2-yl]-N-hydroxy-N-methylpropionamid und dessen Calciumsalz.

6. Verbindung nach Anspruch 1, nämlich 3-[4,5-Bis-(4-methoxyphenyl)-thien-2-yl]-N-ethyl-N-hydroxypropionamid.

7. Verfahren zur Herstellung der Verbindungen von Anspruch 1, dadurch gekennzeichnet, daß man:
- den Ester der Formel II: 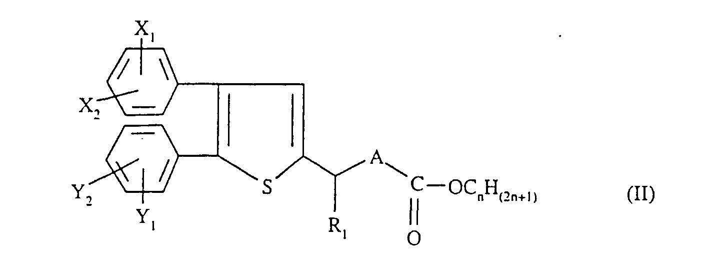 in der X₁, X₂, Y₁, Y₂, R₁ und A die in Anspruch 1 angegebenen Bedeutungen besitzen und n 1 oder 2 darstellt,
- zu der Säure der Formel III verseift: 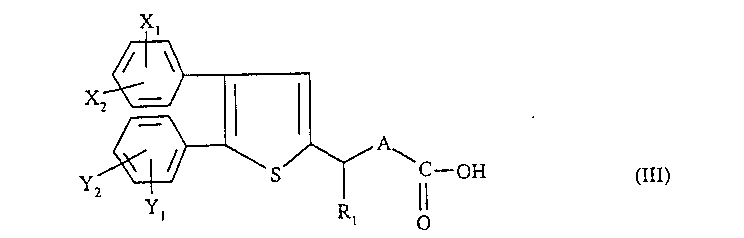 in der X₁, X₂, Y₁, Y₂, R₁ und A die oben angegebenen Bedeutungen besitzen,
- welche Säure in die entsprechende aktivierte Verbindung der Formel (IV) umgewandelt wird: 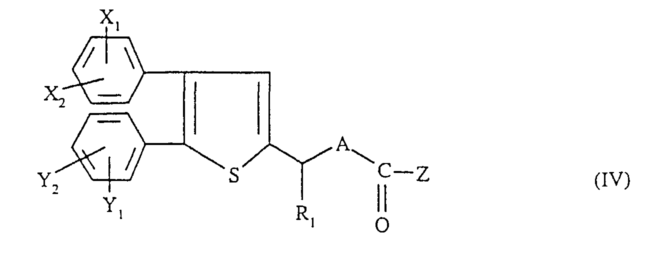 in der:
- X₁, X₂, Y₁, Y₂, R₁ und A die oben angegebenen Bedeutungen besitzen und
- Z ein Chloratom oder einen Imidazol-1-yl-Rest bedeutet,
- welche man mit O-silyliertem Hydroxylamin der Formel V umsetzt: 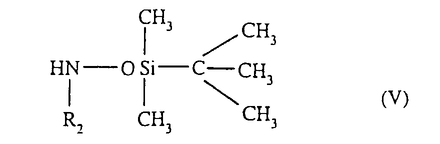 in der R₂ die in Anspruch 1 angegebenen Bedeutungen besitzt,
- zur Bildung der Verbindung der Formel VI: 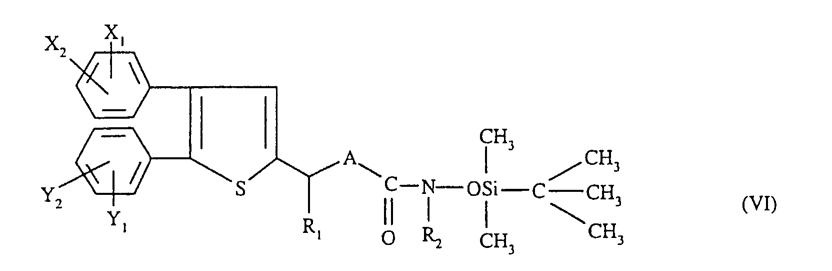 in der X₁, X₂, Y₁, Y₂, A, R₁ und R₂ die oben angegebenen Bedeutungen besitzen,
- welche man hydrolysiert zur Bildung der entsprechenden Verbindung der Formel I,
- und gewünschtenfalls die in dieser Weise erhaltenen Verbindungen in ihre Additionssalze mit pharmazeutisch annehmbaren Basen umwandelt.

8. Verbindung nach einem der Ansprüche 1 bis 6 als Arzneimittel.

9. Pharmazeutische Zubereitungen zur Behandlung von akuten oder chronischen rheumatologischen Entzündungen und Darm- oder Hautentzündungen, bei denen Leukotriene beteiligt sind, enthaltend als Wirkstoff mindestens eine Verbindung nach einem der Ansprüche 1 bis 6 allein oder in Kombination mit einem oder mehreren pharmazeutisch geeigneten Trägermaterialien.

## Claims

1. Thiophene compounds of formula I 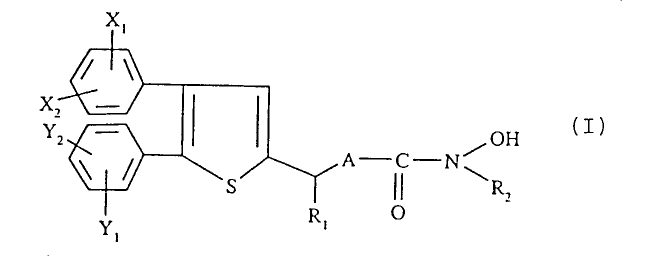 wherein:
- X₁, X₂, Y₁ and Y₂, which are identical or different, each represents a hydrogen or halogen atom, an alkyl or alkoxy radical each having from 1 to 5 carbon atoms in straight or branched chain, or a trifluoromethyl radical;
- R₁ represents a hydrogen atom or an alkyl radical having from 1 to 5 carbon atoms in straight or branched chain;
- A represents a straight hydrocarbon chain having from 1 to 5 carbon atoms each of which carbon atoms is optionally mono- or di-substituted by an alkyl radical having from 1 to 5 carbon atoms, and
- R₂ represents a hydrogen atom, an alkyl radical having from 1 to 5 carbon atoms in straight or branched chain, a cyclohexyl radical or a benzyl radical;
and their physiologically tolerable addition salts with appropriate bases.

2. A compound of claim 1 which is 3-[4,5-bis(4-methoxyphenyl) thien-2-yl] -N-hydroxy-N-methylpropionamide and its calcium salt.

3. A compound of claim 1 which is 3-[4-(4-fluorophenyl)-5- (4-methoxyphenyl) thien-2-yl] -N-hydroxy-N-methylpropionamide.

4. A compound of claim 1 which is 4-[4,5-bis(4-methoxyphenyl) thien-2-yl] -N-hydroxy-N-methylbutanamide.

5. A compound of claim 1 which is 3-[4-(4-methylphenyl)-5- (4-methoxyphenyl)thien-2-yl] -N-hydroxy-N-methylpropionamide and its calcium salt.

6. A compound of claim 1 which is 3-[4,5-bis(4-methoxyphenyl) thien-2-yl] -N-ethyl-N-hydroxypropionamide.

7. The process for the preparation of compounds of claim 1, characterised in that :
- the ester of formula II: 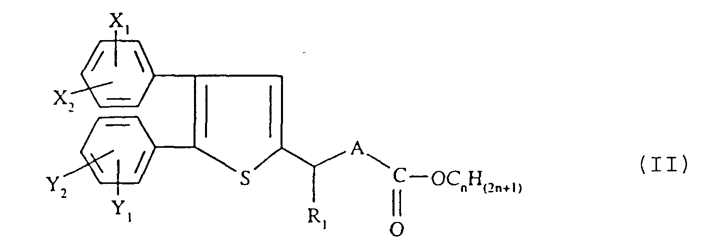 wherein X₁, X₂, Y₁, Y₂, R₁ and A are as defined in claim 1 and n represents 1 or 2 is hydrolysed
- to form an acid of formula III: 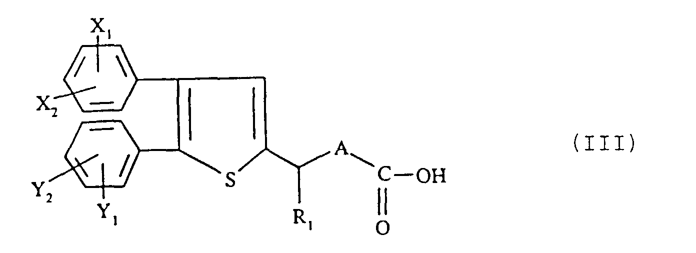 wherein X₁, X₂, Y₁, Y₂, R₁ and A are as defined above,
- which acid is converted into a corresponding activated compound of formula IV : 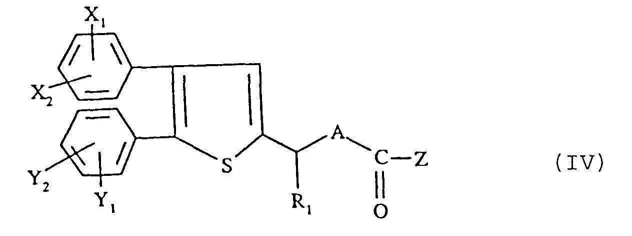 wherein
- X₁, X₂, Y₁, Y₂, R₁ and A are as defined above, and
- Z represents a chlorine atom or an imidazol-1-yl radical,
- with which the O-silylated hydroxylamine of formula V : 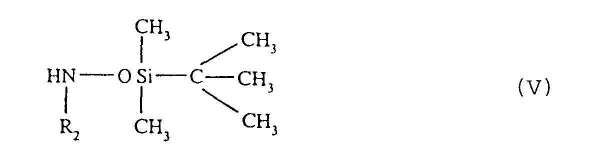 wherein R₂ is as defined in claim 1 is reacted
- to obtain the compound of formula VI : 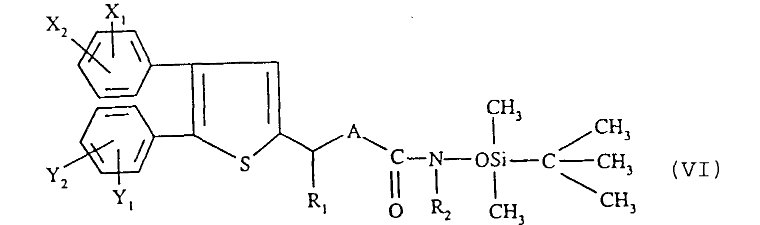 wherein X₁, X₂, Y₁, Y₂, A, R₁ and R₂ are as defined above,
- which is hydrolysed to obtain the corresponding compound of formula I,
- and, if desired, the compounds so obtained are converted into their addition salts with pharmaceutically acceptable bases.

8. As a medicament, a compound according to any one of claims 1 to 6.

9. Pharmaceutical compositions which can be used in the treatment of rheumatological inflammation of an acute or chronic nature and inflammation of the intestinal or cutaneous type in which leucotrienes are found to be implicated, comprising as active ingredient at least one of the compounds according to any one of claims 1 to 6, alone or in combination with one or more appropriate pharmaceutical excipients.
